(19) Europäisches Patentamt
European Patent Office
Office européen des brevets

(11) Veröffentlichungsnummer: **0 473 549 B1**

(12) **EUROPÄISCHE PATENTSCHRIFT**

(45) Veröffentlichungstag der Patentschrift: **25.01.95**

(51) Int. Cl.[6]: **C07C 317/18**, C07C 317/44, C08K 5/36, C09K 15/14

(21) Anmeldenummer: **91810660.0**

(22) Anmeldetag: **20.08.91**

Die Akte enthält technische Angaben, die nach dem Eingang der Anmeldung eingereicht wurden und die nicht in dieser Patentschrift enthalten sind.

# (54) Sulfoxide von Alkylthiomethylphenolen.

(30) Priorität: **28.08.90 CH 2788/90**

(43) Veröffentlichungstag der Anmeldung:
**04.03.92 Patentblatt 92/10**

(45) Bekanntmachung des Hinweises auf die Patenterteilung:
**25.01.95 Patentblatt 95/04**

(84) Benannte Vertragsstaaten:
**DE FR GB IT**

(56) Entgegenhaltungen:
**EP-A- 0 140 298**
**GB-A- 1 396 469**
**JP-A-43 016 741**
**US-A- 4 759 862**
**US-A- 4 857 572**

(73) Patentinhaber: **CIBA-GEIGY AG**
**Klybeckstrasse 141**
**CH-4002 Basel (CH)**

(72) Erfinder: **Meier, Hans-Rudolf, Dr.**
**Route du Confin 54**
**CH-1723 Marly (CH)**
Erfinder: **Dubs, Paul, Dr.**
**Route du Confin 14**
**CH-1723 Marly (CH)**

**EP 0 473 549 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Sulfoxide von Alkylthiomethylphenolen, Mischungen dieser Sulfoxide mit den nicht oxidierten Verbindungen, diese Verbindungen oder Mischungen enthaltende Zusammensetzungen von organischen Materialien, die Verwendung der oben genannten Verbindungen und Mischungen als Stabilisatoren in organischen Materialien, die gegen oxidativen, thermischen und/oder lichtinduzierten Abbau empfindlich sind, sowie ein Verfahren zum Stabilisieren der oben genannten Materialien.

Alkylthiomethylierte Phenole und deren Oxidationsprodukte werden als Stabilisatoren in organischen Polymeren eingesetzt. Siehe z.B. US-A-4,759,862 und US-A-4,857,572. Aus der JP-A-68,16741 sind (3,5-Disubstituierte-4-hydroxybenzyl)sulfoxide als Antioxidantien in Polyolefinen bekannt. Auch in der JP-A-74,27092 werden solche Verbindungen in Polyolefinzusammensetzungen verwendet. In der JP-A-68,3773 werden entsprechende am Schwefelatom dioxidierte Verbindungen beschrieben. Die GB-A-917,370 offenbart Bis(3,5-disubstituierte-4-hydroxybenzyl)sulfoxide als Antioxidantien für organische Materialien. Ferner beschreiben die GB-A-1,396,469 sowie die JP-A-430 16741 (Chem. abstr. vol. 70, no. 77582 $\mu$) weitere am S-Atom oxidierte Alkylthioalkylenphenole als Stabilisatoren.

Im Bereich der Antioxidantien für organische Polymere besteht weiterhin ein Bedarf an wirksamen Verbindungen. Insbesondere im Bereich der Elastomere und Schmierstoffe erfordern neue Anwendungstechniken und neue Zusammensetzungen Anpassungen im Hinblick auf die Stabilisierung der Substrate.

Es wurde nun gefunden, dass bestimmte Sulfoxide von Alkylthiomethylphenolen sehr gut zur Stabilisierung von gegen oxidativen, thermischen und/oder lichtinduzierten Abbau empfindlichen organischen Materialien geeignet sind.

Gegenstand der vorliegenden Erfindung sind Verbindungen der Formel I,

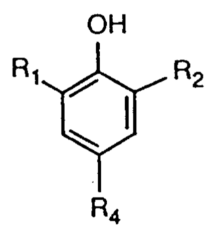

(I),

worin

$R_1$ Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl oder $C_7$-$C_9$-Phenylalkyl bedeutet, $R_2$ für Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl, $C_7$-$C_9$-Phenylalkyl oder einen Rest $-CH_2$-SO-$R_5$ steht, worin $R_5$ $C_8$-$C_{20}$-Alkyl, welches gegebenenfalls durch -O-, -S- oder -SO-Gruppen unterbrochen ist, durch ein bis zwei Hydroxylgruppen substituiertes $C_2$-$C_{12}$-Alkyl, Phenyl, Benzyl, $-(CH_2)_nCOOR_6$ oder $-CH(CH_3)COOR_6$ bedeutet, worin $R_6$ für $C_1$-$C_{20}$-Alkyl steht und n 1 oder 2 ist, und

$R_4$ für Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl, $C_7$-$C_9$-Phenylalkyl oder einen Rest $-CH_2$-SO-$R_5$ steht, worin

$R_5$ $-(CH_2)_nCOOR_6$ oder $-CH(CH_3)COOR_6$ bedeutet, worin $R_6$ die oben angegebene Bedeutung hat,

mit der Massgabe, dass einer der Reste $R_2$, $R_3$ oder $R_4$ für die Gruppe $-CH_2$-SO-$R_5$ steht.

$R_1$, $R_2$, $R_4$ und $R_6$ als $C_1$-$C_{20}$-Alkyl können linear oder verzweigt sein und bedeuten beispielsweise Methyl, Ethyl, n-Propyl, Isopropyl, n-Butyl, tert.-Butyl, n-Pentyl, Isopentyl, n-Hexyl, 2-Ethylhexyl, n-Heptyl, n-Octyl, i-Octyl, tert.-Octyl, Decyl, Dodecyl, Tetradecyl, Octadecyl oder Icosyl. Bevorzugt sind $R_1$ und $R_2$ als Alkyl $C_1$-$C_{18}$-Alkyl, besonders bevorzugt $C_1$-$C_{12}$-Alkyl, insbesondere $C_1$-$C_9$-Alkyl. $R_4$ als Alkyl ist bevorzugt $C_1$-$C_{18}$-Alkyl. $R_6$ als Alkyl ist bevorzugt $C_8$-$C_{18}$-Alkyl, insbesondere $C_{13}$-Alkyl.

$R_5$ als $C_8$-$C_{20}$-Alkyl kann linear oder verzweigt sein und die gleichen Bedeutungen haben wie für $R_1$, $R_2$, $R_4$ und $R_6$ angegeben ist, beginnend mit der entsprechenden Anzahl der C-Atome. Bevorzugte Alkylgruppen $R_5$ sind $C_8$-$C_{12}$-Alkyl.

$R_1$, $R_2$ und $R_4$ als $C_2$-$C_{18}$-Alkenyl können z.B. Vinyl, Allyl, Methallyl, 1,1-Dimethylallyl, Butenyl, Hexenyl, Octenyl, Undecenyl, Dodecenyl oder Octadecenyl sein.

$R_5$ als durch ein bis zwei Hydroxylgruppen substituiertes $C_2$-$C_{12}$-Alkyl kann ein lineares oder verzweigtes substituiertes Alkyl sein und z.B. 2-Hydroxyethyl, 1,2-Dihydroxyethyl, Hydroxy- oder Dihydroxypropyl, -butyl, -pentyl, -hexyl, -heptyl, -octyl, -nonyl, -decyl, -undecyl, -dodecyl, insbesondere 2-Hydroxyethyl oder 2,3-Dihydroxypropyl, bedeuten.

$R_1$, $R_2$ und $R_4$ als $C_5$-$C_7$-Cycloalkyl können Cyclopentyl, Cyclohexyl und Cycloheptyl bedeuten, insbesondere Cyclopentyl oder Cyclohexyl.

$R_1$, $R_2$ und $R_4$ als $C_5$-$C_7$-Cycloalkenyl sind beispielsweise Cyclopentenyl, Cyclohexenyl und Cycloheptenyl, insbesondere Cyclopentenyl oder Cyclohexenyl.

$R_1$, $R_2$ und $R_4$ als $C_7$-$C_9$-Phenylalkyl stehen z.B. für Benzyl, Phenylethyl, $\alpha$-Methylbenzyl, 3-Phenylpropyl, 2-Methyl-phenylethyl, 1-Methyl-phenylethyl oder $\alpha,\alpha$-Dimethylbenzyl, insbesondere für Benzyl.

Von besonderem Interesse sind Verbindungen der Formel I, worin $R_1$ Wasserstoff, $C_1$-$C_{18}$-Alkyl, $C_5$-$C_6$-Cycloalkyl oder $C_5$-$C_7$-Cycloalkenyl bedeutet, $R_2$ für -$CH_2$-SO-$R_5$ steht, $R_4$ Wasserstoff oder $C_1$-$C_{18}$-Alkyl bedeutet, $R_5$ für $C_8$-$C_{20}$-Alkyl, Phenyl, Benzyl oder -$CH_2COOR_6$ steht und $R_6$ $C_1$-$C_{20}$-Alkyl bedeutet.

Ferner sind Verbindungen der Formel I bevorzugt worin $R_1$ Wasserstoff, $C_1$-$C_{18}$-Alkyl, insbesondere $C_1$-$C_{12}$-Alkyl, Cyclopentenyl oder Cyclohexenyl bedeutet, $R_2$ für Wasserstoff, $C_1$-$C_{18}$-Alkyl, insbesondere $C_1$-$C_{12}$-Alkyl, vor allem $C_1$-$C_4$-Alkyl, Cyclopentenyl, Cyclohexenyl oder -$CH_2$-SO-$R_5$ steht, $R_4$ Wasserstoff, $C_1$-$C_{18}$-Alkyl, insbesondere $C_1$-$C_{12}$-Alkyl oder -$CH_2$-SO-$R_5$ bedeutet, $R_5$ für $C_8$-$C_{20}$-Alkyl, Phenyl, Benzyl oder -$CH_2COOR_6$ steht und $R_6$ $C_6$-$C_{20}$-Alkyl ist.

Andere bevorzugte Verbindungen der Formel I sind solche, worin $R_1$ $C_1$-$C_{18}$-Alkyl bedeutet, $R_2$ für $C_1$-$C_{12}$-Alkyl, Cyclopentenyl oder -$CH_2$-SO-$R_5$ steht, $R_4$ $C_1$-$C_{12}$-Alkyl oder -$CH_2$-SO-$R_5$ bedeutet, $R_5$ für $C_8$-$C_{20}$-Alkyl, Phenyl, Benzyl -$CH(CH_3)COOR_6$, -$CH_2CH_2OH$ oder -$CH_2COOR_6$ steht und $R_6$ $C_6$-$C_{20}$-Alkyl ist.

Bevorzugt sind Verbindungen der Formel I, worin $R_1$ Wasserstoff, $C_1$-$C_{18}$-Alkyl, $C_5$-$C_6$-Cycloalkyl oder $C_5$-$C_6$-Cycloalkenyl bedeutet, $R_2$ für -$CH_2$-SO-$R_5$ steht, $R_4$ $C_1$-$C_{18}$-Alkyl, $C_5$-$C_6$-Cycloalkyl oder $C_5$-$C_6$-Cycloalkenyl bedeutet und $R_5$ für $C_8$-$C_{20}$-Alkyl, Phenyl, Benzyl oder -$CH_2COOR_6$ steht.

Weiterhin bevorzugt sind Verbindungen der Formel I, worin $R_1$ Wasserstoff, $C_1$-$C_{12}$Alkyl oder $C_5$-$C_6$-Cycloalkyl bedeutet und $R_5$ für $C_8$-$C_{18}$-Alkyl, Phenyl, Benzyl oder -$CH_2COOR_6$ steht.

Von besonderem Interesse sind auch Verbindungen der Formel I, worin $R_1$ $C_1$-$C_9$-Alkyl bedeutet und $R_5$ für $C_8$-$C_{12}$-Alkyl, Phenyl oder Benzyl steht, insbesondere Verbindungen, worin $R_5$ $C_8$-$C_{12}$-Alkyl bedeutet.

Ausserdem sind Verbindungen der Formel I bevorzugt, worin $R_1$ und $R_2$ unabhängig voneinander $C_1$-$C_{18}$-Alkyl, $C_5$-$C_6$-Cycloalkyl oder $C_5$-$C_6$-Cycloalkenyl bedeuten, und $R_4$ für einen Rest -$CH_2$-SO-$R_5$ steht, worin $R_5$ -$(CH_2)_nCOOR_6$ ist.

Besonders interessant sind auch Verbindungen der Formel I, worin $R_4$ für einen Rest -$CH_2$-SO-$R_5$ steht und $R_5$ -$(CH_2)_nCOOR_6$ ist.

Bevorzugt sind auch Verbindungen der Formel I, worin $R_1$ und $R_2$ unabhängig voneinander $C_1$-$C_{12}$-Alkyl, $C_5$-$C_6$-Cycloalkyl oder $C_5$-$C_6$-Cycloalkenyl bedeuten und $R_5$ für -$(CH_2)_nCOOR_6$ steht.

Besonders bevorzugt sind Verbindungen der Formel I, worin $R_1$ $C_1$-$C_9$-Alkyl bedeutet und $R_5$ für $C_8$-$C_{12}$-Alkyl, Phenyl oder Benzyl steht, insbesondere solche Verbindungen, worin $R_5$ für $C_8$-$C_{12}$-Alkyl steht.

Besonders zweckmässig sind Verbindungen der Formel I, worin $R_2$ für -$CH_2$-SO-$R_5$ steht.

Ein weiterer Gegenstand der Erfindung sind Mischungen von Verbindungen der Formel I mit Verbindungen der Formel III

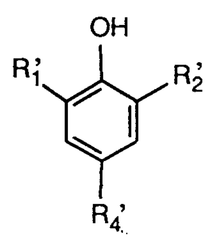 (III),

worin

$R_1$' Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl oder $C_7$-$C_9$-Phenylalkyl bedeutet, $R_2$' für Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl, $C_7$-$C_9$-Phenylalkyl oder einen Rest -$CH_2$-S-$R_5$ steht, worin $R_5$ $C_8$-$C_{20}$-Alkyl, welches gegebenenfalls durch -O-, -S- oder -SO-Gruppen unterbrochen ist, durch ein bis zwei Hydroxylgruppen substituiertes $C_2$-$C_{12}$-Alkyl, Phenyl, Benzyl, -$(CH_2)_nCOOR_6$ oder -$CH(CH_3)COOR_6$ bedeutet, worin $R_6$ für $C_1$-$C_{20}$-Alkyl steht und n 1 oder 2 ist, und $R_4$' für Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl, $C_7$-$C_9$-Phenylalkyl oder einen Rest -$CH_2$-S-$R_5$ steht, worin $R_5$ -$(CH_2)_nCOOR_6$ oder -$CH(CH_3)COOR_6$ bedeutet, worin $R_6$ die oben angegebene Bedeutung hat, mit der Massgabe, dass nur einer der Reste $R_2$', $R_3$' oder $R_4$' für die Gruppe -$CH_2$-S-$R_5$ steht.

Bevorzugte Mischungen sind solche, in welchen sich die Verbindungen der Formel I von jenen der Formel III nur dadurch unterscheiden, dass letztere an Stelle der -SO-Gruppierungen -S-Gruppen enthaltend, sonst aber die gleiche Struktur besitzen wie erstere.

Interessant sind Mischungen, die durch partielle Oxidation von mindestens einer Verbindung der Formel III erhältlich sind.

Von besonderem Interesse sind Mischungen, worin das Verhältnis der Verbindungen der Formel III zu jenen der Formel I 1:9 bis 9:1, insbesondere 1:9 bis 1:1, beträgt.

Ein weiterer Gegenstand der Erfindung sind Zusammensetzungen enthaltend ein gegen oxidativen, thermischen und/oder lichtinduzierten Abbau empfindliches organisches Material und mindestens eine Verbindung der Formel I oder mindestens eine Mischung von Verbindungen der Formel I mit Verbindungen der Formel III, sowie die Verwendung der genannten Verbindungen und Mischungen zum Stabilisieren von gegen oxidativen, thermischen und/oder lichtinduzierten Abbau empfindlichen organischen Materialien.

Bevorzugt sind Zusammensetzungen, die mindestens eine Verbindung der Formel I und ein gegen oxidativen, thermischen und/oder lichtinduzierten Abbau empfindliches organisches Material enthalten.

Als Beispiele von derartigen organischen Materialien, die in den erfindungsgemässen Zusammensetzungen enthalten sind und die erfindungsgemäss stabilisiert werden können, seien beispielsweise die folgenden erwähnt:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen, ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

3a. Kohlenwasserstoffharze (z.B. $C_5$-$C_9$) inklusive hydrierte Modifikationen davon (z.B. Klebrigmacherharze) und Mischungen von Polyalkylenen und Stärke.

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder a-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die Zusammensetzungen enthalten zweckmässig 0,01-10 Gew.-% der erfindungsgemässen Verbindungen bzw. Mischungen, bezogen auf das organische Material, insbesondere 0,05-5,0 Gew.-%, vorzugsweise 0,05-3 Gew.-%, z.B. 0,1-2 Gew.-%.

Bei dem gegen oxidativen, thermischen und/oder lichtinduzierten Abbau empfindlichen Material kann es sich also beispielsweise um ein organisches Polymer, einen Schmierstoff oder eine Hydraulikflüssigkeit handeln. Bei den organischen Polymeren handelt es sich dabei vorzugsweise um synthetische Polymere, insbesondere um Elastomere.

Besonders bevorzugt sind Zusammensetzungen, die Elastomere oder Schmierstoffe und mindestens eine Verbindung der Formel I oder II oder Mischungen von mindestens einer Verbindung der Formel I bzw. II mit mindestens einer Verbindung der Formel III enthalten, sowie die Verwendung dieser Verbindungen und Mischungen als Stabilisatoren für die genannten Materialien.

Ein weiterer Gegenstand der Erfindung ist ein Verfahren zum Stabilisieren von gegen oxidativen, thermischen und/oder lichtinduzierten Abbau empfindlichen organischen Materialien, dadurch gekennzeichnet, dass man eine Verbindung der Formel I bzw. II oder Mischungen von mindestens einer Verbindung der Formel I bzw. II mit mindestens einer Verbindung der Formel III in diese Materialien einarbeitet oder auf diese aufbringt.

Die in Frage kommenden Schmierstoffe basieren beispielsweise auf mineralischen oder synthetischen Oelen oder Mischungen davon. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Oele und Fette, beispielsweise basierend auf einem Mineralöl. Bevorzugt sind Oele.

Eine weitere Gruppe von Schmierstoffen, die zur Anwendung gelangen können, sind pflanzliche oder tierische Oele, Fette, Talge und Wachse oder deren Gemische untereinander oder Gemische mit den erwähnten mineralischen oder synthetischen Oelen. Pflanzliche und tierische Oele, Fette, Talge und Wachse sind beispielsweise Palmkernöl, Palmöl, Olivenöl, Rueböl, Rapsöl, Leinöl, Erdnussöl, Sojabohnenöl, Baumwollöl, Sonnenblumenöl, Kürbiskernöl, Kokosnussöl, Maisöl, Rizinusöl, Baumnussöl und Mischungen davon, Fischöle, Talge von Schlachttieren wie Rindertalg, Klauenfett und Knochenöl sowie deren modifizierte, epoxidierte und sulfoxidierte Formen, beispielsweise epoxidiertes Sojabohnenöl.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylenoxide, der Phosphorsäureester, der Poly-$\alpha$-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropantricaprylat oder Gemische davon, eines Tetraesters von Pentaerythrit mit einer einwenigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Capryl- und Sebacinsäure oder von einem Gemisch davon. Besonders geeignet sind neben Mineralölen z.B. Poly-$\alpha$-Olefine, Schmierstoffe auf Esterbasis, Phosphate, Glykole, Polyglykole und Polyalkylenglykole, sowie deren Mischungen mit Wasser.

Als Elastomere können die erfindungsgemässen Zusammensetzungen beispielsweise folgende Materialien enthalten:

1. Polydiene, wie beispielsweise Polybutadien, Polyisopren oder Polychloropren; Blockpolymere, wie beispielsweise Styrol/Butadien/Styrol, Styrol/Isopren/Styrol oder Acrylnitril/Butadien-Copolymere.

2. Copolymere von Mono- und Diolefinen untereinander oder mit anderen Vinylmonomeren, wie z.B. Aethylen-Alkylacrylat-Copolymere, Aethylen-Alkylmethacrylat-Copolymere, Aethylen-Vinylacetat-Copolymere sowie Terpolymere von Aethylen mit Propylen und einem Dien, wie Hexadien, Dicyclopentadien oder Aethylidennorbornen.
3. Halogenhaltige Polymere, wie z.B. Polychloropren, Chlorkautschuk, chloriertes oder chlorsulfoniertes Polyäthylen, Epichlorhydrin-Homo- und -Copolymere, Chlortrifluoroäthylen-Copolymere, Polymere aus halogenhaltigen Vinylverbindungen, wie z.B. Polyvinylidenchlorid, Polyvinylidenfluorid; sowie deren Copolymere, wie Vinylchlorid-Vinylidenchlorid, Vinylchlorid-Vinylacetat oder Vinylidenchlorid-Vinylacetat.
4. Polyurethane, die sich von Polyäthern, Polyestern und Polybutadien mit endständigen Hydroxylgruppen einerseits und aliphatischen oder aromatischen Polyisocyanaten andererseits ableiten sowie deren Vorprodukte.
5. Naturkautschuk.
6. Mischungen (Polyblends) der vorgenannten Polymeren.
7. Wässrige Emulsionen natürlicher oder synthetischer Kautschuke, wie z.B. Naturkautschuk-Latex oder Latices von carboxylierten Styrol-Butadien-Copolymeren.

Gegebenenfalls liegen diese Elastomere als Latices vor und können als solche stabilisiert werden.

Bevorzugt sind Zusammensetzungen, welche als Elastomeres ein Polydien, wie Polybutadien-Kautschuk, ein halogenhaltiges Polymer, wie Polyvinylidenfluorid, oder ein Polyurethan enthalten.

Die Einarbeitung in die organischen Materialien kann beispielsweise durch Einmischen der erfindungsgemässen Verbindungen oder Mischungen und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemässen Verbindungen bzw. Mischungen in Polymere besteht in deren Zugabe vor oder während der Polymerisation der entsprechenden Monomeren bzw. vor der Vernetzung. Im Falle der Zugabe vor oder während der Polymerisation können die Verbindungen der Formel I oder II bzw. die erfindungsgemässen Mischungen aus Verbindungen der Formeln I bzw. II mit Verbindungen der Formel III auch als Regulatoren für die Kettenlänge der Polymerisation (Kettenabbrecher) wirken.

Die erfindungsgemässen Verbindungen oder Mischungen können auch in Form eines Masterbatches, der diese Verbindungen beispielsweise in einer Konzentration von 2,5 bis 25 Gew.-% enthält, den zu stabilisierenden Kunststoffen zugesetzt werden.

Erfindungsgemässe Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Erfindungsgemässe Schmierstoffzusammensetzungen finden z.B. Verwendung in Verbrennungsmotoren, z.B. in Kraftfahrzeugen.

Zusätzlich zu den erfindungsgemässen Verbindungen bzw. Mischungen können die erfindungsgemässen Zusammensetzungen, insbesondere wenn sie organische, vorzugsweise synthetische, Polymere enthalten, noch weitere übliche Additive enthalten. Beispiele für solche Additive sind:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Ditert.butyl-4-i-butylphenol, 2,6-Dicyclopentyl-4-methylphenol, 2-($\alpha$-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert.-butylphenol, 2,4-Dioctylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol, 2,6-Di-tert.-butyl-hydrochinon, 2,5-Di-tert.-butyl-4-hydroxyanisol,3,5-Di-tert.-butyl-4-hydroxyanisol, 3,5-Di-tert-butyl-4-hydroxyphenyl-stearat, Bis-(3,5-di-tert.butyl-4-hydroxyphenyl)adipat.

1.4. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol), 4,4'-Thio-bis-(3,6-di-sec.-amylphenol), 4,4'-Bis-(2,6-dimethyl-4-hydroxyphenyl)-disulfid.

1.5. Alkyliden-Bisphenole, z.B. 2,2'-Methylen-bis-(6-tert.butyl-4-methylphenol), 2,2'-Methylen-bis-(6-tert.butyl-4-ethylphenol),2,2'-Methylen-bis-[4-methyl-6-(α-methylcyclohexyl)-phenol], 2,2'-Methylen-bis-(4-methyl-6-cyclohexylphenol), 2,2'-Methylenbis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'tert-butyl-4'-hydroxyphenyl)-butyrat],Bis-(3-tert.butyl-4-hydroxy-5-methyl-phenyl)-dicyclopentadien, Bis-[2-(3'-tert.butyl-2'-hydroxy-5'-methyl-benzyl)-6-tert.butyl-4-methyl-phenyl]-terephthalat, 1,1-Bis-(3,5-dimethyl-2-hydroxyphenyl)-butan, 2,2-Bis-(3,5-di-tert.-butyl-4-hydroxyphenyl)-propan, 2,2-Bis-(5-tert.-butyl-4-hydroxy-2-methyl-phenyl)-4-n-dodecylmercapto-butan, 1,1,5,5-Tetra-(5-tert.-butyl-4-hydroxy-2-methylphenyl)-pentan.

1.6. O-, N- und S-Benzylverbindungen, z.B. 3,5,3',5'-Tetra-tert.-butyl-4,4'-dihydroxydibenzylether, Octadecyl-4-hydroxy-3,5-dimethylbenzyl-mercaptoacetat, Tris-(3,5-ditert.-butyl-4-hydroxybenzyl)-amin, Bis-(4-tert.-butyl-3-hydroxy-2,6-dimethylbenzyl)-dithioterephthalat, Bis-(3,5-di-tert.-butyl-4-hydroxybenzyl)-sulfid, Isooctyl-3,5-di-tert.-butyl-4-hydroxybenzyl-mercaptoacetat.

1.7. Hydroxybenzylierte Malonate, z.B. Dioctadecyl-2,2-bis-(3,5-di-tert.butyl-2-hydroxybenzyl)-malonat, Di-octadecyl-2-(3-tert.-butyl-4-hydroxy-5-methylbenzyl)-malonat, Didodecylmercaptoethyl-2,2-bis-(3,5-di-tert.-butyl-4-hydroxybenzyl)-malonat, Di-[4-(1,1,3,3-tetramethylbutyl)-phenyl]-2,2-bis-(3,5-di-tert.-butyl-4-hydroxybenzyl)-malonat.

1.8. Hydroxybenzyl-Aromaten, z.B. 1,3,5-Tris-(3,5-di-tert.-butyl-4-hydroxybenzyl)-2,4,6-trimethylbenzol, 1,4-Bis-(3,5-di-tert.-butyl-4-hydroxybenzyl)-2,3,5,6-tetramethylbenzol, 2,4,6-Tris-(3,5-di-tert.-butyl-4-hydroxybenzyl)-phenol.

1.9. Triazinverbindungen, z.B. 2,4-Bis-octylmercapto-6-(3,5-di-tert.butyl-4-hydroxyanilino)-1,3,5-triazin, 2-Octylmercapto-4,6-bis-(3,5-di-tert.-butyl-4-hydroxyanilino)1,3,5-triazin, 2-Octylmercapto-4,6-bis-(3,5-di-tert.-butyl-4-hydroxyphenoxy) 1,3,5-triazin, 2,4,6-Tris-(3,5-di-tert.-butyl-4-hydroxyphenoxy)-1,2,3-triazin, 1,3,5-Tris-(3,5-ditert.-butyl-4-hydroxybenzyl)-isocyanurat, 1,3,5-Tris-(4-tert.-butyl-3-hydroxy-2,6-dimethylbenzyl)-isocyanurat, 2,4,6-Tris-(3,5-di-tert.-butyl-4-hydroxyphenylethyl)-1,3,5-triazin,1,3,5-Tris-(3,5-di-tert.butyl-4-hydroxyphenylpropionyl)-hexahydro-1,3,5-triazin, 1,3,5-Tris-(3,5-dicyclohexyl-4-hydroxybenzyl)-isocyanurat.

1.10. Benzylphosphonate, z.B. Dimethyl-2,5-di-tert.butyl-4-hydroxybenzylphosphonat, Diethyl-3,5-di-tert.-butyl-4-hydroxybenzylphosphonat, Dioctadecyl-3,5-di-tert.-butyl-4-hydroxybenzylphosphonat, Dioctadecyl-5-tert.butyl-4-hydroxy-3-methylbenzylphosphonat, Ca-Salz des 3,5-Di-tert.-butyl-4-hydroxybenzyl-phosphonsäure-monoethylesters.

1.11. Acylaminophenole, z.B. 4-Hydroxy-laurinsäureanilid, 4-Hydroxystearinsäureanilid, N-(3,5-di-tert.butyl-4-hydroxyphenyl)-carbaminsäureoctylester.

1.12. Ester der β-(3,5-Di-tert.butyl-4-hydroxyphenyl)-propionsäure mit ein- oder mehrwenigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.13. Ester der β-(5-tert.Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(3,5-Dicyclohexyl-4-hydroxyphenyl)-propionsäure mit ein- oder mehrwenigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der 3,5-Di-tert.-butyl-4-hydroxyphenylessigsäure mit ein- oder mehrwenigen Alkoholen, wie z.B. mit Methanol, Ethanol, Octadecanol, 1,6-Hexandiol, 1,9-Nonandiol, Ethylenglycol, 1,2-Propandiol,

Neopentylglycol, Thiodiethylenglycol, Diethylenglycol, Triethylenglycol, Pentaerythrit, Tris-(hydroxy)-ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.-butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. 5'-Methyl-, 3',5'-Di-tert.butyl-, 5'-tert.-Butyl-, 5'-(1,1,3,3-Tetramethylbutyl)-, 5-Chlor-3',5'-di-tert.butyl, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-, Mischung aus 5-Chlor-3'-tert.-butyl-5'-(2-octyloxycarbonylethyl)- und 5-Chlor-3'-tert.-butyl-5'-[2-(2-ethylhexyloxy)-carbonylethyl],5-Chlor-3'-tert.-butyl-5'-(2-methoxycarbonylethyl), 3'-tert.-Butyl-5'-(2-methoxycarbonylethyl)-, 3'-tert.-Butyl-5'-(2-octyloxycarbonylethyl), 3'-tert.-Butyl-5'-[2-(2-ethylhexyloxy)-carbonylethyl]-, 3'-Dodecyl-5'-methyl- und 3'-tert.-Butyl-5'-(2-isooctyloxycarbonylethyl)-2'-hydroxyphenyl-2H-benztriazol(2), 2,2'-Methylen-bis[4-(1,1,3,3-tetramethylbutyl)-6-benztriazol-2-yl-phenol]; Umesterungsprodukt von 2-[3'-tert.-Butyl-5'-(2-methoxycarbonylethyl)-2'-hydroxy-phenyl]-2H-benztriazol mit Polyethylenglycol 300;

$$[R\text{-}CH_2CH_2\text{-}COO(CH_2)_3]_2\!-$$

mit R = 3'-tert.-Butyl-4'-hydroxy-5'-2H-benzotriazol-2-yl-phenyl.

2.2. 2-Hyroxybenzophenone, wie z.B. das 4-Hydroxy-, 4-Methoxy-, 4-Octoxy-, 4-Decyloxy-, 4-Dodecyloxy, 4-Benzyloxy-, 4,2',4'-Trihydroxy, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butyl-benzoyl)-resorcin, Benzoylresorcin, 3,5-Di-tert.butyl-4-hydroxybenzoesäure-2,4-di-tert.-butylphenylester, 3,5-Di-tert.butyl-4-hydroxybenzoesäurehexadecylester, 3,5-Di-tert.-butyl-4-hydroxybenzoesäure-octadecylester, 3,5-Di-tert.-butyl-4-hydroxybenzoesäure-2-methyl-4,6-di-tert.-butylphenylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert.butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethylpiperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert.-butylbenzyl)-malonat, 3-n-Octyl-7,7,9,9-tetramethyl-1,3,8-triazasprio[4.5]-decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6 di-(4-n-butylamino- 1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro-[4.5]-decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Di-octyloxy-5,5'-di-tert.-butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten

Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazi n, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid N,N'-Diacetal-adipinsäure-dihydrazid, N,N'-Bis-salicyloyl-oxalsäure-dihydrazid, N,N'-Bis-saticyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Bis-(2,6-di-tert.-butyl-4-methylphenyl)-pentaerythritdiphosphit, Bis-isodecyloxy-pentaerythritdiphosphit, Bis-(2,4-di-tert.-butyl-6-methylphenyl)-pentaerythritdiphosphit, Bis-(2,4,6-tri-tert.-butylphenyl)-pentaerythritdiphosphit, Tristearyl-sorbit-triphosphit, Tetrakis-(2,4-di-tert.butylphenyl)-4,4'-biphenylen-diphosphonit, 6-Isooctyloxy-2,4,8,10-tetra-tert.-butyl-12H-dibenz[d,g]-1,3,2-dioxaphosphocin, 6-Fluor-2,4,8,10-tetra-tert.-butyl-12-methyl-dibenz[d,g]-1,3,2-dioxaphosphocin.

5. Peroxidzerstörende Verbindungen, wie z.B. Ester der $\beta$-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-($\beta$-dodecylmercapto)-propionat.

6. Polyamidstabilisatoren, wie z.B Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfit, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Handelt es sich bei den erfindungsgemässen Zusammensetzungen um solche auf Basis von Schmierstoffen und Hydraulikflüssigkeiten, können diese ebenfalls weitere Additive enthalten, die zugegeben werden, um gewisse Gebrauchseigenschaften zu verbessern, wie z.B. weitere Antioxidantien, Metalldesaktivatoren, Rostinhibitoren, Viskositäts-Index-Verbesserer, Stockpunkterniedriger, Dispergiermittel/Tenside und Verschleissschutzadditive.

Beispiele für Antioxidantien sind der weiter oben wiedergegebenen Auflistung unter dem Titel "1. Antioxidantien", insbesondere Punkte 1.1 bis 1.10 zu entnehmen. Beispiele für weitere zusätzliche Additive sind die folgenden:

Beispiele für aminische Antioxidantien:

N,N'-Di-isopropyl-p-phenylendiamin, N,N'-Di-sec-butyl-p-phenylendiamin, N,N'-Bis-(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methylphenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, 2,3-Dihydro-3,3-dimethyl-4H-1,4-benzothiazin, Phenothiazin, N-Allylphenothiazin.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure.

Beispiele für Metall-Desäktivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol,5,5'-Methylenbisbenztriazol, 4,5,6,7-Tetrahydrobenztriazol, Salicyliden-propylendiamin, Salicylaminoguanidin und dessen Salze.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Alkenylbernsteinsäureanhydrid, z.B. Dodecenylbernsteinsäure-anhydrid, Alkenylbernsteinsäure-Teilester und -Teilamide, 4-Nonylphenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.:
Substituierte Imidazoline und Oxazoline.
c) Phosphorhaltige Verbindungen, z.B.:
Aminsalze von Phosphorsäurepartialestern oder Phosphonsäurepartialestern, Zinkdialkyldithiophosphate.
d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

Beispiele für Verschleissschutz-Additive sind:

Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate, Diethanolaminomethyltolyltriazol, Di(2-ethylhexyl)aminomethyltolyltriazol.

Die Sulfoxide der vorliegenden Erfindung der Formel I bzw. II können z.B. durch Oxidation von Verbindungen der Formel III erhalten werden. Als Oxidationsmittel kommen z.B. Wasserstoffperoxid oder Percarbonsäuren, organische Hydroperoxide oder andere geeignete organische oder anorganische Oxidationsmittel in Frage, wobei nach in der Literatur einschlägig bekannten Methoden vorgegangen werden kann. Geeignete Percarbonsäuren sind z.B. m-Chlorperbenzoesäure, Peressigsäure oder Trifluorperessigsäure. Als Hydroperoxide lassen sich beispielsweise tert-Butylhydroperoxid oder Cumolperoxid verwenden.

Die Oxidation der Ausgangsprodukte kann auch mit Oxidationsmitteln, die in situ hergestellt werden, erfolgen. Solche Oxidationsmittel sind z.B. Percarbonsäuren, welche im Gemisch aus Wasserstoffperoxid und Carbonsäuren entstehen.

Das Oxidationsmittel wird, je nach gewünschtem Produkt, zweckmässig in stöchiometrischer Menge oder im Überschuss zugegeben. Dabei werden die Reaktionsparameter so gewählt, dass eine möglichst geringe Aufoxidation der Sulfoxide zu Sulfonen erfolgt. Die Oxidation wird zweckmässig in Gegenwart von Lösungsmitteln ausgeführt. Als Lösungsmittel dienen z.B. nicht oxidierbare organische Lösungsmittel wie z.B. chlorierte Kohlenwasserstoffe, Ketone oder Kohlenwasserstoffe. Geeignete chlorierte Kohlenwasserstoffe sind beispielsweise Methylenchlorid oder Chloroform, geeignete Ketone können z.B. Aceton, Methylethylketon oder Methylisopropylketon sein, als Kohlenwasserstoffe eignen sich insbesondere aromatische Kohlenwasserstoffe, beispielsweise Toluol oder Xylol.

Die Reaktionstemperatur kann beispielsweise zwischen -40 und +80°C liegen. Die Reaktionszeiten liegen je nach Temperatur und spezifischer Reaktion beispielsweise zwischen 10 Minuten und 24 Stunden.

Die Ausgangsprodukte, die Verbindungen der Formel III und deren Herstellung sind literaturbekannt und in zahlreichen Patenten, z.B. US-A 4,759,862 und US-A 4,857,572, beschrieben oder können nach dort beschriebenen Verfahren analog hergestellt werden.

Die Herstellung der erfindungsgemässen Mischungen aus Verbindungen der Formel I bzw. II und solchen der Formel III kann z.B. durch Mischen der Einzelverbindungen im gewünschten Mengenverhältnis erfolgen. Bevorzugt werden diese Mischungen aber durch partielle Oxidation von Verbindungen der Formel III z.B. nach den oben angegebenen Methoden erhalten, wobei man das Oxidationsmittel in unterstöchiometrischer Menge einsetzt. Seine Menge richtet sich nach dem gewünschten Gehalt an Verbindung der Formel I bzw. II oder III in der Mischung. Sie kann durch einfache Vorversuche bzw. Berechnung leicht ermittelt werden.

Nähere Details können den nachfolgenden Herstellungsbeispielen entnommen werden.

Die nachfolgenden Beispiele erläutern die Erfindung weiter. Angaben in Teilen oder Prozenten beziehen sich, ebenso wie in der übrigen Beschreibung und in den Patentansprüchen, auf das Gewicht, sofern nichts anderes angegeben ist.

Beispiele 1 - 7

0.05 mol des jeweiligen substituierten Thiomethylphenols der Formel III werden, je nach Löslichkeit von Edukt und Produkt, in Aceton oder Methylenchlorid gelöst. In 5-10 Minuten werden bei 0-20 °C 0.05 mol 30%iges Wasserstoffperoxid zugetropft. Anschliessend wird während 4-20 Stunden bei Raumtemperatur gerührt. Die Vollständigkeit der Reaktion wird durch Dünnschichtchromatographie überprüft. Danach wird das Lösungsmittel unter vermindertem Druck abdestilliert. Die Produkte werden durch Chromatographie an Kieselgel in einem Eluens in reiner Form erhalten.
Eine Auflistung der Produkte der Beispiele 1 - 7 befindet sich in Tabelle 1. Tabelle 2 gibt die charakteristischen $^1$H-Kernresonanz-Daten wieder.

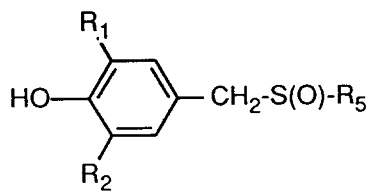

Tabelle 1

| Beispiel Nr. | $R_1$ | $R_2$ | $R_5$ | Schmelzpunkt [°C] | Eluens |
|---|---|---|---|---|---|
| 1 (a) | t-Butyl | t-Butyl | $CH_2COOC_8H_{17}$* | Oel | B |
| 2 | t-Butyl | t-Butyl | $CH_2COOC_{13}H_{27}$* | Oel | E |
| 3 | t-Butyl | t-Butyl | $CH_2COOC_{18}H_{37}$ | 62 | B |
| 4 | Methyl | Cyclopent-3-enyl | $CH_2COOC_8H_{17}$* | Oel | A |
| 5 | t-Butyl | t-Butyl | $CH_2$COO-EH** | Oel | F |
| 6 (b) | t-Butyl | t-Butyl | $CH(CH_3)$COO-EH** | Oel | F |
| 7 (b) | Methyl | t-Butyl | $CH(CH_3)$COO-EH** | Oel | F |

* Isomerengemisch
**2-Ethylhexyl
(a) Einsatz von 0,1 mol Wasserstoffperoxid
(b) Lösungsmittel Essigsäure (Aufarbeitung: zuerst Waschen mit Natriumhydrogencarbonatlösung)
Elutionsmittel:
A Methylenchlorid/Essigester (49:1)
B Methylenchlorid/Essigester (19:1)
E Methylenchlorid
F Hexan/Essigester (7:1)

Tabelle 2

Charakteristische $^1H$-NMR-Signale der Verbindungen der Beispiele 1 - 7 gemessen in Deuterochloroform bei 100 MHz (Standard Tetramethylsilan)

| Beispiel-Nr. | Aryl$C\underline{H}_2$-SO | | SO$C\underline{H}_2$COO | |
|---|---|---|---|---|
| | δ [ppm] | $J_{AB}$[Hz] | δ[ppm] | $J_{AB}$[Hz] |
| 1 | 4.19/4.02 | 13.5 | 3.64/3.49 | 13.5 |
| 2 | 4.16/4.04 | 12.5 | 3.61/3.51 | 12.5 |
| 3 | 4.15/4.04 | 12.5 | 3.61/3.51 | 12.5 |
| 4 (*) | 4.14/3.97 | 13.5 | 3.64/3.48 | 13.5 |
| 5 (**) | 4.19/4.02 | 13 | 3.64/3.51 | 12 |
| 6 | 4.05/3.88 | 13 | (***) | - |
| 7 | 4.04/3.89 | 12.5 | (****) | - |

* bei 300 MHz,
** bei 250 MHz
*** 3 Quartette ($^3J_{HH}=7$) bei 3.69/3.47 und 3.35 ($C\underline{H}CH_3$) Diastereomerengemisch
**** 3 Quartette ($^3J_{HH}=7$) bei 3.70/3.48 und 3.35 ($C\underline{H}CH_3$) Diastereomerengemisch

Beispiel 8:

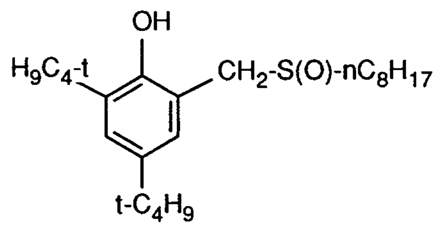

Zu 18.2 g 2,4-Bis-tert.-butyl-6octylthiomethyl-phenol, gelöst in 120 ml Aceton, wird bei 0 °Ceine äquimolare Menge 30%iges Wasserstoffperoxid getropft. Nach 26 stündigem Rühren bei Raumtemperatur wird das Lösungsmittel unter vermindertem Druck abdestilliert. Chromatographie an Kieselgel mit Hexan/Essigester (9:1) als Elutionsmittel ergibt 11.95 g 2,4-Bistert.-butyl-6-octylsulfinylmethyl-phenol vom Schmelzpunkt 58-59 °C.

| | | | | |
|---|---|---|---|---|
| Elementaranalyse: | ber.: | C 72.58 %<br>H 10.59 %<br>S 8.42 % | gef.: | C 72.58 %<br>H 10.29 %<br>S 8.49 % |

Charakteristische $^1$H-Kernresonanz-Signale: 4.31 und 3.86 ppm ($J_{AB}$ = 14 Hz).

Beispiel 9:

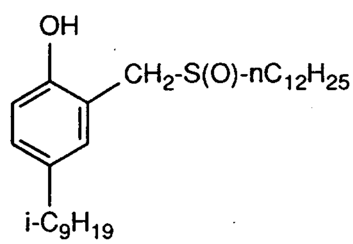

Analog zu dem unter Beispiel 8 beschriebenen Verfahren werden 8.17 g 2-Dodecylthiomethyl-4-nonylphenol, welches durch Alkylthiomethylierung von technischem 4-Nonylphenol (Isomerengemisch) mit Formaldehyd und 1-Dodecanthiol erhalten wird, mit Wasserstoffperoxidlösung umgesetzt. Nach Säulenchromatographie mit Hexan/Essigester (4:1) werden 6.7 g 2-Dodecylsulfinylmethyl-4-nonylphenol als farbloses Oel isoliert.

| | | | | |
|---|---|---|---|---|
| Elementaranalyse: | ber.: | C 74.61 %<br>H 11.18 %<br>S 7.11 % | gef.: | C 74,67 %<br>H 11.10 %<br>S 7.14 % |

Charakteristische $^1$H-Kernresonanz-Signale: ~4.45 und ~3.80 ppm ($J_{AB}$ = 14 Hz)

Beispiel 10:

Werden analog zu Beispiel 1 statt eines zweifachen Ueberschusses nur 1.1 Aequivalente Wasserstoffperoxid eingesetzt, so erhält man nach Eindampfen des Reaktionsgemisches ohne weitere Trennung ein oranges Harz, welches gemäss $^1$H-NMR ein Gemisch aus dem Ausgangsthioether und dem Sulfoxid im Verhältnis 1:2 darstellt.

Beispiel 11:

Werden analog zu Beispiel 1 statt eines zweifachen Ueberschusses 0.5 Aequivalente Wasserstoffperoxid eingesetzt, so erhält man nach Eindampfen des Reaktionsgemisches ohne weitere Trennung ein oranges viskoses Oel, welches gemäss $^1$H-NMR ein Gemisch aus dem Ausgangsthioether und dem Sulfoxid im Verhältnis 2:1 darstellt.

Beispiele 12 - :

Die Verbindungen der Beispiele 12 - werden analog zu den Verbindungen der Beispiele 1 - 7 hergestellt.
Die Verbindungen sind in der Tabelle 5a dargestellt, ihre charakteristischen $^1$H-Kernresonanzdaten sind der Tabelle 6 zu entnehmen. Angegeben sind die Verschiebungswerte (δ) in [ppm] bezogen auf Tetramethylsilan. Als Lösungsmittel diente Deuterochloroform.

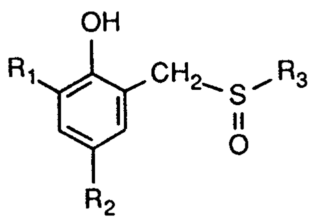

Tabelle 5a:

| Bsp. Nr. | $R_1$ | $R_2$ | $R_3$ | Schmelz-punkt [°C] | Eluens |
|---|---|---|---|---|---|
| 12 | 2-$C_{18}H_{37}$[1)] | Methyl | $C_{12}H_{25}$ | gelbl. Flüssigkeit | F |
| 13 | 2-$C_{18}H_{37}$[1)] | Methyl | t-$C_{12}H_{25}$[3)] | gelbl. Oel | B |
| 14 | 2-$C_{18}H_{37}$[1)] | Methyl | $C_8H_{17}$ | gelbl. Oel | B |
| 15 | 2-$C_{18}H_{37}$[1)] | Methyl | t-$C_8H_{17}$[4)] | farbloses Oel | B |
| 16 | t-$C_8H_{17}$[2)] | Methyl | $C_{12}H_{25}$ | gelbl. Oel | D*) |
| 17 | t-$C_8H_{17}$[2)] | Methyl | $C_8H_{17}$ | gelbl. viskoses Oel | B*) |
| 18 | t-$C_8H_{17}$[2)] | Methyl | $CH_2COO^iC_8H_{17}$[5)] | gelbes Oel | E |
| 19 | t-$C_8H_{17}$[2)] | Methyl | $CH_2COO^iC_{13}H_{27}$[6)] | gelbl. Oel | E |
| 20 | t-Butyl | t-Butyl | $C_{12}H_{25}$ | gelbes Oel | B |
| 21 | t-Butyl | t-Butyl | t-$C_{12}H_{25}$[3)] | gelbl. Harz | B |
| 22 | t-Butyl | t-Butyl | 2-$C_8H_{17}$[7)] | gelbl. Harz | B |
| 23 | t-Butyl | t-Butyl | t-$C_9H_{19}$[7)] | oranges Harz | B |
| 24 | t-Butyl | t-Butyl | $CH_2COO^iC_8H_{17}$[5)] | gelbes Oel | C |
| 25 | t-Butyl | t-Butyl | $CH_2COO^iC_{13}H_{27}$[6)] | gelbl. Harz | A |
| 26 | Methyl | Methyl | $C_{12}H_{25}$ | gelbl. Flüssigkeit | B |
| 27 | Methyl | Methyl | t-$C_{12}H_{25}$[3)] | gelbl. Harz | B |
| 28 | Methyl | Methyl | t-$C_9H_{19}$[7)] | gelbl. viskoses Oel | A |
| 29 | Methyl | Methyl | $CH_2COO^iC_{13}H_{27}$[6)] | gelbes Oel | B |

A Hexan/Essigester 49:1
B Hexan/Essigester 9:1
C Hexan/Aceton 8:1
D Hexan/Methylenchlorid 1:1
E Hexan/Aceton 9:1
F Hexan/Essigester 4:1
G Hexan/Essigester 7:3
1) 2-$C_{18}H_{37}$: n-Octadecan-2-yl
2) t-$C_8H_{17}$: 1,1,3,3-Tetramethylbutyl
3) t-$C_{12}H_{25}$: Isomerengemisch aus 1,1,3,3,5,5-Hexamethylhexyl und 2,2,4,6,6-Pentamethylhept-4-yl
4) $CH_2COO^iC_8H_{17}$: Ester aus prim. Octanol-Isomerengemisch
5) Isomerengemisch
6) 2-$C_8H_{17}$: n-Octan-2-yl
7) t-$C_9H_{19}$: Isomerengemisch
*) Nachreinigung durch Säulenchromatographie
**) umkristallisiert aus Aceton

Tabelle 6

| Bsp. Nr. | $ArCH_2SO$ $\delta$ [ppm] | Kopplungskonstante [Hz] des AB-Systems |
|---|---|---|
| 12 | 4.35 4.32 3.80 3.77 | 14 |
| 13 | 4.40-4.20 (m) und 3.4-3.55 (m) a) | |
| 14 | 4.35 4.32 3.82 3.79 | 14 |
| 15 | 4.24 3.49 3.48 b) | 13 |
| 16 | 4.34 3.76 | 14 |
| 17 | 4.35 3.76 | 14 |
| 18 | 4.59 3.97 | 14 |
| 19 | 4.60 3.97 | 14 |
| 20 | 4.30 3.92 | 14 |
| 21 | 4.3-4.45 (m) und 3.35-3.55 (m) b) | |
| 22 | 3.9-4.3 (m) b) | |
| 23 | 4.2-4.45 (m) und 3.35-3.55 (m) b) | |
| 24 | 4.59 4.08 | 14 |
| 25 | 4.60 4.08 | 14 |
| 26 | 4.35 3.50 | 14 |
| 27 | 4.15-4.35 (m) und 3.4-3.6 (m) b) | |
| 28 | 4.15-4.40 (m) und 3.4-3.55 (m) b) | |
| 29 | 4.56 4.01 | 14 |

a) Diastereomerengemisch
b) Isomerengemisch
(m) Multiplett

Beispiel 30:

Stabilisierung von Polybutadien-Kautschuk (Brabender Test)
100 Teile Polybutadien, welches mit 0,36 % 2,6-Di-tert-butyl-p-cresol vorstabilisiert ist, werden zusätzlich mit 0,25 % des zu prüfenden Stabilisators in einem Brabender Plastographen bei 160 °C und 60 Umdrehungen pro Minute während 30 Minuten geknetet.

Das Gerät arbeitet nach folgendem Prinzip:
Zwei Knetwalzen, die in einer thermostatisch beheizten Knetkammer rotieren, werden von einem pendelnd gelagerten Motor angetrieben. Zur Ueberwindung des Fliesswiderstandes des Kunststoffes, der sich in der Knetkammer befindet, ist ein bestimmtes Drehmoment erforderlich, das mit dem Drehmomentspendel zur Anzeige gebracht wird und zeitabhängig mitgeschrieben wird. Aus dem Verlauf der Drehmomentskurve wird die Induktionszeit ermittelt, d.h. die Knetzeit in Minuten bis zum Anstieg des Drehmoments um 100 mp nach dem Drehmomentminimum. Die Versuche zeigen, dass durch den zugesetzten Stabilisator der Fliesswiderstand wesentlich länger konstant gehalten wird, der Kunststoff also länger beständig ist.
Die Ergebnisse sind in Tabelle 5 aufgeführt.

Tabelle 5

| Verbindung aus Beispiel-Nr. | Brabender Induktionszeit [min] |
|---|---|
| Kontrolle* | 9.5 |
| 1 | >30 |
| 2 | >30 |
| 3 | >30 |
| 4 | >30 |
| 5 | >30 |

*die Kontrolle enthält keinen erfindungsgemässen Stabilisator

**Patentansprüche**

1. Verbindungen der Formel I

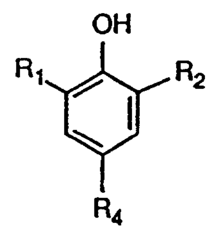

(I),

worin
$R_1$ Wasserstoff, $C_1$-$C_{20}$-Alkyl, $C_2$-$C_{18}$-Alkenyl $C_5$-$C_7$-Cycloalkyl, $C_5$-$C_7$-Cycloalkenyl, Phenyl der $C_7$-$C_9$-Phenylalkyl bedeutet $R_2$ für Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl, $C_7$-$C_9$-Phenylalkyl der einen Rest $-CH_2$-SO-$R_5$ steht, worin $R_5$ $C_8$-$C_{20}$-Alkyl, welches gegebenenfalls durch -O-, -S- oder -SO-Gruppen unterbrochen ist, durch ein zwei Hydroxylgruppen substituiertes $C_2$-$C_{12}$-Alkyl, Phenyl, Benzyl, $-(CH_2)_nCOOR_6$ oder $-CH(CH_3)COOR_6$ bedeutet, worin $R_6$ für $C_1$-$C_{20}$-Alkyl steht und n 1 oder 2 ist und
$R_4$ für Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl, $C_7$-$C_9$-Phenylalkyl oder einen Rest $-CH_2$-SO-$R_5$ steht, worin
$R_5$ $-(CH_2)_nCOOR_6$ oder $-CH(CH_3)COOR_6$ bedeutet, worin $R_6$ die oben angegebene Bedeutung hat,
mit der Massgabe, dass einer der Reste $R_2$ oder $R_4$ für die Gruppe $-CH_2$-SO-$R_5$ steht.

2. Verbindungen nach Anspruch 1, worin $R_1$ Wasserstoff, $C_1$-$C_{18}$-Alkyl, $C_5$-$C_6$-Cycloalkyl oder $C_5$-$C_7$-Cycloalkenyl bedeutet, $R_2$ für $-CH_2$-SO-$R_5$ steht, $R_4$ Wasserstoff oder $C_1$-$C_{18}$-Alkyl bedeutet, $R_5$ für $C_8$-$C_{20}$-Alkyl, Phenyl, Benzyl oder $-CH_2COOR_6$ steht und $R_6$ $C_1$-$C_{20}$-Alkyl bedeutet.

**3.** Verbindungen nach Anspruch 1, worin $R_1$ $C_1$-$C_{18}$-Alkyl, $R_2$ für $-CH_2$-SO-$R_5$ steht, $R_4$ $C_1$-$C_{18}$-Alkyl, insbesondere $C_1$-$C_{12}$-Alkyl, bedeutet, $R_5$ für $C_8$-$C_{20}$-Alkyl, Phenyl, Benzyl $-CH(CH_3)COOR_6$, $-CH_2CH_2OH$ oder $-CH_2COOR_6$ steht und $R_6$ $C_6$-$C_{20}$-Alkyl ist.

**4.** Verbindungen, nach Anspruch 1, worin $R_1$ Wasserstoff, $C_1$-$C_{18}$-Alkyl, insbesondere $C_1$-$C_{12}$-Alkyl, $C_5$-$C_6$-Cycloalkyl oder $C_5$-$C_6$-Cycloalkenyl bedeutet, $R_2$ für $-CH_2$-SO-$R_5$ steht, $R_4$ $C_1$-$C_{18}$-Alkyl, $C_5$-$C_6$-Cycloalkyl oder $C_5$-$C_6$-Cycloalkenyl bedeutet und $R_5$ für $C_8$-$C_{20}$-Alkyl, insbesondere $C_8$-$C_{18}$-Alkyl, Phenyl, Benzyl oder $-CH_2COOR_6$ steht.

**5.** Verbindungen nach Anspruch 4, worin $R_1$ $C_1$-$C_9$-Alkyl bedeutet und $R_5$ für $C_8$-$C_{12}$-Alkyl, Phenyl oder Benzyl, insbesondere für $C_8$-$C_{12}$-Alkyl steht.

**6.** Verbindungen nach Anspruch 1, worin $R_1$ und $R_2$ unabhängig voneinander $C_1$-$C_{18}$-Alkyl, insbesondere $C_1$-$C_{12}$-Alkyl, vor allem $C_1$-$C_9$-Alkyl, $C_5$-$C_6$-Cycloalkyl oder $C_5$-$C_6$-Cycloalkenyl bedeuten und $R_4$ für einen Rest $-CH_2$-SO-$R_5$ steht, worin $R_5$ $-(CH_2)_nCOOR_6$ ist.

**7.** Mischungen von mindestens einer der in den Ansprüchen 1 bis 6 definierten Verbindungen mit mindestens einer Verbindung der Formel III

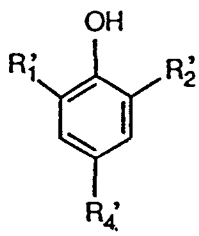 (III),

worin

$R_1$' Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl oder $C_7$-$C_9$-Phenylalkyl bedeutet, $R_2$' für Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl, $C_7$-$C_9$-Phenylalkyl oder einen Rest $-CH_2$-S-$R_5$ steht, worin $R_5$ $C_8$-$C_{20}$-Alkyl, welches gegebenenfalls durch -O-, -S- oder -SO-Gruppen unterbrochen ist, durch ein bis zwei Hydroxylgruppen substituiertes $C_2$-$C_{12}$-Alkyl, Phenyl, Benzyl, $-(CH_2)_nCOOR_6$ oder $-CH(CH_3)COOR_6$ bedeutet, worin $R_6$ für $C_1$-$C_{20}$-Alkyl steht und n 1 oder 2 ist, und $R_4$' für Wasserstoff, $C_1$-$C_{20}$-Alkyl, $C_2$-$C_{18}$-Alkenyl, $C_5$-$C_7$-Cycloalkyl, $C_5$-$C_7$-Cycloalkenyl, Phenyl, $C_7$-$C_9$-Phenylalkyl oder einen Rest $-CH_2$-S-$R_5$ steht, worin $R_5$ $-(CH_2)_nCOOR_6$ oder $-CH(CH_3)COOR_6$ bedeutet, worin $R_6$ die oben angegebene Bedeutung hat, mit der Massgabe, dass nur einer der Reste $R_2$', $R_3$' oder $R_4$' für die Gruppe $-CH_2$-S-$R_5$ steht.

**8.** Mischungen nach Anspruch 7, erhältlich durch partielle Oxidation mindestens einer Verbindung der Formel III.

**9.** Mischungen nach Anspruch 7 oder 8, worin das Verhältnis der Verbindungen der Formel III zu jenen der Formel I 1:9 bis 9:1, insbensondere 1:9 bis 1:1, beträgt.

**10.** Zusammensetzungen enthaltend ein gegen oxidativen, thermischen und/oder lichtinduzierten Abbau empfindliches organisches Material und mindestens eine Verbindung der Formel I nach Anspruch 1 oder mindestens eine in Anspruch 7 definierte Mischung.

**11.** Zusammensetzung nach Anspruch 10, worin das organische Material ein organisches, vorzugsweise synthetisches, Polymer, ein Schmierstoff oder eine Hydraulikflüssigkeit, insbesondere ein Elastomer oder ein Schmierstoff, ist.

**12.** Verwendung von Verbindungen der Formel I nach Anspruch 1 oder von Mischungen nach Anspruch 7 als Stabilisatoren in einem gegen oxidativen, thermischen und/oder lichtinduzierten Abbau empfindlichen organischen Material. ..

**13.** Verwendung nach Anspruch 12, worin das organische Material ein organisches, vorzugsweise synthetisches, Polymer, ein Schmierstoff oder eine Hydraulikflüssigkeit, insbesondere ein Elastomer oder ein Schmierstoff, ist.

**14.** Verfahren zum Stabilisieren von gegen oxidativen, thermischen und/oder lichtinduzierten Abbau empfindlichen Materialien, dadurch gekennzeichnet, dass man mindestens eine Verbindung der Formel I nach Anspruch 1 oder Mischungen nach Anspruch 7 in diese Materialien einarbeitet oder auf diese aufbringt.

## Claims

**1.** A compound of the formula I

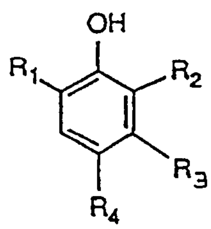

(I),

in which
$R_1$ is hydrogen, $C_1$-$C_{20}$alkyl, $C_2$-$C_{18}$alkenyl, $C_5$-$C_7$cycloalkyl, $C_5$-$C_7$cycloalkenyl, phenyl or $C_7$-$C_9$phenylalkyl, $R_2$ is hydrogen, $C_1$-$C_{20}$alkyl, $C_2$-$C_{18}$alkenyl, $C_5$-$C_7$cycloalkyl, $C_5$-$C_7$cycloalkenyl, phenyl, $C_7$-$C_9$phenylalkyl or a radical $-CH_2$-SO-$R_5$, in which $R_5$ is $C_8$-$C_{20}$alkyl which may be interrupted by -O-, -S- or -SO- groups, mono- or dihydroxysubstituted $C_2$-$C_{12}$alkyl, phenyl, benzyl, $-(CH_2)_nCOOR_6$ or $-CH(CH_3)COOR_6$, in which $R_6$ is $C_1$-$C_{20}$alkyl and n is 1 or 2, and $R_4$ is hydrogen, $C_1$-$C_{20}$alkyl, $C_2$-$C_{18}$alkenyl, $C_5$-$C_7$cycloalkyl, $C_5$-$C_7$cycloalkenyl, phenyl, $C_7$-$C_9$phenylalkyl or a radical $-CH_2$-SO-$R_5$, in which $R_5$ is $-(CH_2)_nCOOR_6$ or $-CH(CH_3)COOR_6$, in which $R_6$ is as defined above, with the proviso that one of the radicals $R_2$ or $R_4$ is the group $-CH_2$-SO-$R_5$.

**2.** A compound according to claim 1, in which $R_1$ is hydrogen, $C_1$-$C_{18}$alkyl, $C_5$-$C_6$cycloalkyl or $C_5$-$C_7$cycloalkenyl, $R_2$ is $-CH_2$-SO-$R_5$, $R_4$ is hydrogen or $C_1$-$C_{18}$alkyl, $R_5$ is $C_8$-$C_{20}$alkyl, phenyl, benzyl or $-CH_2COOR_6$ and $R_6$ is $C_1$-$C_{20}$alkyl.

**3.** A compound according to claim 1, in which $R_1$ is $C_1$-$C_{18}$alkyl, $R_2$ is $-CH_2$-SO-$R_5$, $R_4$ is $C_1$-$C_{18}$alkyl, especially $C_1$-$C_{12}$alkyl, $R_5$ is $C_8$-$C_{20}$alkyl, phenyl, benzyl, $-CH(CH_3)COOR_6$, $-CH_2CH_2OH$ or $-CH_2COOR_6$ and $R_6$ is $C_6$-$C_{20}$alkyl.

**4.** A compound according to claim 1, in which $R_1$ is hydrogen, $C_1$-$C_{18}$alkyl, especially $C_1$-$C_{12}$alkyl, $C_5$-$C_6$cycloalkyl or $C_5$-$C_6$cycloalkenyl, $R_2$ is $-CH_2$-SO-$R_5$, $R_4$ is $C_1$-$C_{18}$alkyl, $C_5$-$C_6$cycloalkyl or $C_5$-$C_6$cycloalkenyl, and $R_5$ is $C_8$-$C_{20}$alkyl, especially $C_8$-$C_{18}$alkyl, phenyl, benzyl or $-CH_2COOR_6$.

**5.** A compound according to claim 4, in which $R_1$ is $C_1$-$C_9$alkyl and $R_5$ is $C_8$-$C_{12}$alkyl, phenyl or benzyl, especially $C_8$-$C_{12}$alkyl.

**6.** A compound according to claim 1, in which $R_1$ and $R_2$, independently of one another, are $C_1$-$C_{18}$alkyl, especially $C_1$-$C_{12}$alkyl, in particular $C_1$-$C_9$alkyl, $C_5$-$C_6$cycloalkyl or $C_5$-$C_6$cycloalkenyl and $R_4$ is a radical $-CH_2$-SO-$R_5$, in which $R_5$ is $-(CH_2)_nCOOR_6$.

**7.** A mixture of at least one of the compounds defined in claims 1 to 6 with at least one compound of the formula III

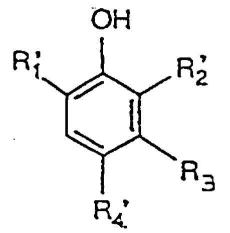

(III),

in which

$R_1$ 'is hydrogen, $C_1$-$C_{20}$alkyl, $C_2$-$C_{18}$alkenyl, $C_5$-$C_7$cycloalkenyl, phenyl or $C_7$-$C_9$phenylalkyl, $R_2$' is hydrogen, $C_1$-$C_{20}$alkyl, $C_2$-$C_{18}$alkenyl, $C_5$-$C_7$cycloalkyl, $C_5$-$C_7$cycloalkenyl, phenyl, $C_7$-$C_9$phenylalkyl or a radical $-CH_2-S-R_5$, in which $R_5$ is $C_8$-$C_{20}$alkyl which may be interrupted by -O-, -S-or -SO- groups, mono- or dihydroxy-substituted $C_2$-$C_{12}$alkyl, phenyl, benzyl, $-(CH_2)_nCOOR_6$ or $-CH(CH_3)COOR_6$, in which $R_6$ is $C_1$-$C_{20}$alkyl and n is 1 or 2, and $R_4$' is hydrogen, $C_1$-$C_{20}$alkyl, $C_2$-$C_{18}$alkenyl, $C_5$-$C_7$cycloalkyl, $C_5$-$C_7$cycloalkenyl, phenyl, $C_7$-$C_9$phenylalkyl or a radical $-CH_2-S-R_5$, in which $R_5$ is $-(CH_2)_nCOOR_6$ or $-CH(CH_3)COOR_6$, in which $R_6$ is as defined above, with the proviso that only one of the radicals $R_2$' or $R_4$' is the group $-CH_2-S-R_5$.

**8.** A mixture according to claim 7, obtainable by partial oxidation of at least one compound of the formula III.

**9.** A mixture according to claim 7 or 8, in which the ratio of the compounds of the formula III to those of the formula I is 1:9 to 9:1, especially 1:9 to 1:1.

**10.** A composition comprising an organic material which is sensitive to oxidative, thermal and/or light-induced degradation and at least one compound of the formula I according to claim 1 or at least one mixture defined in claim 7.

**11.** A composition according to claim 10, in which the organic material is an organic, preferably synthetic, polymer, a lubricant or a hydraulic fluid, especially an elastomer or a lubricant.

**12.** Use of a compound of the formula I according to claim 1 or of a mixture according to claim 7 as stabilizer in an organic material which is sensitive to oxidative, thermal and/or light-induced degradation.

**13.** Use according to claim 12, in which the organic material is an organic, preferably synthetic, polymer, a lubricant or a hydraulic fluid, especially an elastomer or a lubricant.

**14.** A process for stabilizing a material which is sensitive to oxidative, thermal and/or light-induced degradation, which comprises incorporating at least one compound of the formula I according to claim 1 or a mixture according to claim 7 in this material or applying it thereto.

**Revendications**

**1.** Composés de formule I

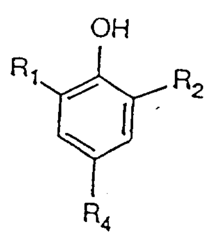

(I),

dans laquelle

$R^1$ signifie les hydrogène, alkyle en $C_1$-$C_{20}$, alcényle en $C_2$-$C_{18}$, cycloalkyle en $C_5$-$C_7$, cycloalcényle en $C_5$-$C_7$, phényle ou phénylalkyle en $C_7$-$C_9$, $R_2$, signifie les hydrogène, alkyle en $C_1$-$C_{20}$,

alcényle en $C_2$-$C_{18}$, cycloalkyle en $C_5$-$C_7$, cycloalcényle en $C_5$-$C_7$, phényle, phényalkyle en $C_7$-$C_9$ ou un reste -$CH_2$-SO-$R_5$, dans lequel $R_5$ signifie les alkyle en $C_8$-$C_{20}$, lequel éventuellement se termine par des groupes -O-, -S- ou -SO-, alkyle en $C_2$-$C_{12}$ substitué par un ou deux groupes hydroxyle, les phényle, benzyle, -$(CH_2)_n$$COOR_6$ ou -$CH(CH_3)COOR_6$, dans lequel $R_6$ signifie les alkyle en $C_1$-$C_{20}$ et n vaut 1 ou 2 et $R_4$ signifie les hydrogéne, alkyle en $C_1$-$C_{20}$, alcényle en $C_2$-$C_{18}$, cycloalkyle en $C_5$-$C_7$, cycloalcényle en $C_5$-$C_7$, phényle, phénylalkyle en $C_7$-$C_9$ ou un reste -$CH_2$-SO-$R_5$ dans lequel $R_5$ signifie -$(CH_2)_n$$COOR_6$ ou -$CH(CH_3)COOR_6$ dans lequel $R_6$ à la signification donnée ci-dessus,

étant entendu que l'un des restes $R_2$ ou $R_4$ représente le groupe -$CH_2$-SO-$R_5$.

**2.** Composés selon la revendication 1, dans lesquelles $R_1$ signifie les hydrogène, alkyle en $C_1$-$C_{18}$, cycloalkyle en $C_5$-$C_6$ ou cycloalcényle en $C_5$-$C_7$, $R_2$ signifie -$CH_2$-SO-$R_5$, $R_4$ signifie les hydrogène ou alkyle en $C_1$-$C_{18}$, $R_5$ signifie les alkyle en $C_8$-$C_{20}$, phényle, benzyle ou -$CH_2COOR_6$ et $R_6$ signifie les alkyle en $C_1$-$C_{20}$.

**3.** Composés selon le revendication 1, dans lesquels $R_1$ signifie les alkyle en $C_1$-$C_{18}$, $R_2$ signifie -$CH_2$-SO-$R_5$, $R_4$ signifie alkyle en $C_1$-$C_{18}$, en particulier alkyle en $C_1$-$C_{12}$, $R_5$ signifie alkyle en $C_8$-$C_{20}$, phényle, benzyle, -$CH(CH_3)COOR_6$, -$CH_2CH_2OH$ ou -$CH_2COOR_6$ et $R_6$ est alkyle en C6-$C_{20}$.

**4.** Composés selon la revendication 1, dans lesquels $R_1$ signifie les hydrogène, alkyle en $C_1$-$C_{18}$, en particulier alkyle en $C_1$-$C_{12}$, cycloalkyle en $C_5$-$C_6$ ou cycloalcényle en $C_5$-$C_6$, $R_2$ signifie -$CH_2$-SO-$R_5$, $R_4$ signifie alkyle en $C_1$-$C_{18}$,cycloalkyle en $C_5$-$C_6$ ou cycloalcényle en $C_5$-$C_6$ et $R_5$ signifie les alkyle en $C_8$-$C_{20}$, en particulier alkyle en $C_8$-$C_{18}$, phényle, benzyle ou -$CH_2COOR_6$.

**5.** Composés selon la revendication 4 dans lesquels $R_1$ signifie les alkyle en $C_1$-$C_9$ et $R_5$ signifie les alkyle en $C_8$-$C_{12}$, phényle ou benzyle, en particulier alkyle $C_8$-$C_{12}$.

**6.** Composés selon la revendication 1, dans lesquels $R_1$ et $R_2$ signifient indépendemment l'un de l'autre alkyle en $C_1$-$C_{18}$, en particulier alkyle en $C_1$-$C_{12}$, surtout alkyle en $C_1$-$C_9$, cycloalkyle en $C_5$-$C_6$ ou cycloalcényle en $C_5$-$C_6$ et $R_4$ signifie un reste -$CH_2$-SO-$R_5$ dans lequel $R_5$ est -$(CH_2)_n$$COOR_6$.

**7.** Mélanges d'au moins un des composé définis dans les revendications 1 à 6 avec au moins un composé de formule III

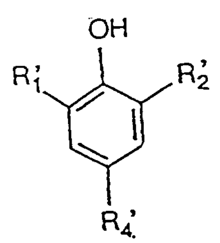

, (III)

dans laquelle

R'$_1$ signifie les hydrogène, alkyle en $C_1$-$C_{20}$, alcényle en $C_2$-$C_{18}$, cycloalkyle en $C_5$-$C_7$, cycloalcényle en $C_5$-$C_7$, phényle ou phényalkyle en $C_7$-$C_9$, $R_2$ représente les hydrogène, alkyle en $C_1$-$C_{20}$, alcényle en $C_2$-$C_{18}$, cycloalkyle en $C_5$-$C_7$, cycloalcényle en $C_5$-$C_7$, phényle, phényalkyle en $C_7$-$C_9$ ou un reste -$CH_2$-S-$R_5$, dans lequel $R_5$ représente alkyle en $C_5$-$C_{20}$, lequel éventuellement est interrompu par des groupes -O-, -S-, ou -SO-, alkyle en $C_2$-$C_{12}$ substitué par un ou deux groupes hydroxyle, phényle, benzyle, -$(CH_2)_n$$COOR_6$, ou -$CH(CH_3)COOR_6$, dans lequel $R_6$ représente les alkyle en $C_1$-$C_{20}$ et n vaut 1 ou 2 et R'$_4$ représente les hydrogène, alkyle en $C_1$-$C_{20}$, alcényle en $C_2$-$C_{18}$, cycloalkyle en $C_5$-$C_7$, cycloalcényle en $C_5$-$C_7$, phényle, phénylalkyle en $C_7$-$C_9$ ou un reste -$CH_2$-S-$R_5$, dans lequel $R_5$ signifie -$(CH_2)_n$$COOR_6$ ou -$CH(CH_3)COOR_6$, dans lequel $R_6$ a la signification donnée ci-dessus, étant entendu que seulement l'un des restes R'$_2$, R'$_3$ ou R'$_4$ représente le groupe -$CH_2$-S-$R_5$.

**8.** Mélanges selon la revendication 7, obtenu par oxydation partielle d'au moins un dérivé de formule III.

**9.** Mélanges selon les revendications 7 ou 8 dans lesquels la proportion de composé de formule III par rapport à ceux de formule I s'éléve de 1:9 à 9:1, en particulier de 1:9 à 1:1.

**10.** Composition contenant un matériau organique sensible aux dégradations oxydantes, thermiques et/ou induites par la lumière et au moins un composé de formule I selon la revendication 1 ou au moins un mélange défini dans la revendication 7.

**11.** Composition selon la revendication 10 dans laquelle le matériau organique est un polymère organique, de préférence synthétique, un lubrifiant ou un fluide hydraulique, en paticulier un élastomère ou un lubrifiant.

**12.** Utilisation des dérivés de formule I selon la revendication 1 ou des mélanges selon la revendication 7 en tant que stabilisant dans un matériau organique sensible au dégradation oxydante, thermiques et/ou induites par la lumière.

**13.** Utilisation selon la revendication 12, dans laquelle le matériau organique est un polymère organique de préférence synthétique, un lubrifiant ou un fluide hydraulique, en particulier un élastomère ou un lubrifiant.

**14.** Procédé pour stabiliser les matériaux sensibles aux dégradations oxydantes, thermiques et/ou induites par la lumière, caractérisé en ce qu'on y incorpore ou y dépose au moins un composé de formule I selon la revendication 1 ou les mélanges selon la revendication 7.